# EUROPEAN PATENT APPLICATION

(11) **EP 4 647 492 A1**
(43) Date of publication of application: **12.11.2025**
(21) Application number: 23914815.8
(22) Date of filing: 27.12.2023
(51) Int. Cl.: C12N 5/071, C12M 3/00

(54) **METHOD FOR CULTURING MUSCLE-DERIVED CELLS, AND CULTURING DEVICE CONTAINING MUSCLE-DERIVED CELLS**

(30) Priority: 06.01.2023 JP 2023001378
(71) Applicant: Integriculture Inc., Tokyo 113-0033 (JP)
(72) Inventor: LU, Mengxue, Tokyo 113-0033 (JP); SHIDA, Miharu, Tokyo 113-0033 (JP); KAWASHIMA, Ikko, Tokyo 113-0033 (JP)
(74) Representative: Grünecker Patent- und Rechtsanwälte PartG mbB
(86) International application number: PCT/JP2023/047124
(87) International publication number: WO 2024/147330

(57) **Abstract**

The present invention aims to provide a novel method for culturing muscle-derived cells. **In** the method, muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells are co-cultured. Alternatively, muscle-derived cells may be cultured in the presence of a supernatant obtained from culturing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

## Description

### [Cross-Reference to Related Literature]

The present application claims priority based on Japanese Patent Application No. 2023-001378 filed on January 6, 2023, which is incorporated by reference herein.

### [Technical Field]

The present invention relates to a method for culturing muscle-derived cells and to a culture apparatus containing muscle-derived cells.

### [Background Art]

Conventionally, standard culture media for culturing muscle cells have been DMEM or RPMI-1640 containing 10% to 20% serum (for example, Scientific Reports volume 12, Article number: 827 (2022)).

### [Summary of the Invention]

### [Problems to be Solved by the Invention]

The present invention aims to provide a method for culturing muscle-derived cells and a culture apparatus containing muscle-derived cells.

### [Means for Solving the Problems]

An aspect of the present invention is a cell culture method comprising the step of co-culturing muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

Another aspect of the present invention is a cell culture method comprising the step of circulating a culture medium between a first culture and a second culture container in a culture apparatus, the first culture container and the second culture container being connected, the first culture container comprising muscle-derived cells and the second culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells,. The second culture container may comprise one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells. The cell culture method may comprise the step of circulating the culture medium among the first culture container, the second culture container and a third culture container in a culture apparatus, the first culture container, the second culture container and the third culture container being connected, the first culture container comprising muscle-derived cells, the second culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, and the third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells are connected. The cell culture method may further comprise the step of circulating the culture medium between the first culture container and the second culture container and the step of circulating the culture medium between the first culture container and a third container in a culture apparatus, wherein the first culture container comprising muscle-derived cells, the second culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, and the third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells are connected.

A further aspect of the present invention is a cell culture apparatus comprising a first culture container and a second culture container, the first culture container and the second culture container being connected so that a culture medium is circulated between the first culture container and the second culture container, the first culture container comprising muscle-derived cells, the second culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells. The apparatus may further comprise a third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, wherein the first to third culture containers are connected so that the culture medium circulates among the first to third culture containers. The apparatus may further comprise a third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, wherein the first culture container and the second culture container are connected so that the culture medium communicates between the first culture container and the second culture container, and the first culture container and the third culture container are connected so that the culture medium communicates between the first culture container and the third culture container.

A further aspect of the present invention is a culture system for co-culturing muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

A further aspect of the present invention is a cell culture method comprising the step of culturing muscle-derived cells in the presence of a culture supernatant obtained by culturing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells. The culture supernatant may further comprise a culture supernatant obtained by culturing muscle-derived cells. The culture for obtaining the culture supernatant may be a co-culture.

A further aspect of the present invention is a culture system for culturing muscle-derived cells in the presence of a culture supernatant obtained by culturing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells. The culture supernatant may further comprise a culture supernatant obtained by culturing muscle-derived cells. The culture for obtaining the culture supernatant may be a co-culture.

A further aspect of the present invention is a system for use in culturing cells, comprising a cell culture container for proliferating the cells and an apparatus for obtaining a culture supernatant comprised in a culture medium for culturing the cells, the apparatus being for co-culturing a plurality of cell types. The apparatus may comprise a plurality of cell culture containers, and some or all of the plurality of cell culture containers may be connected so that the culture supernatant circulates within the containers.

### [Brief Description of the Drawings]

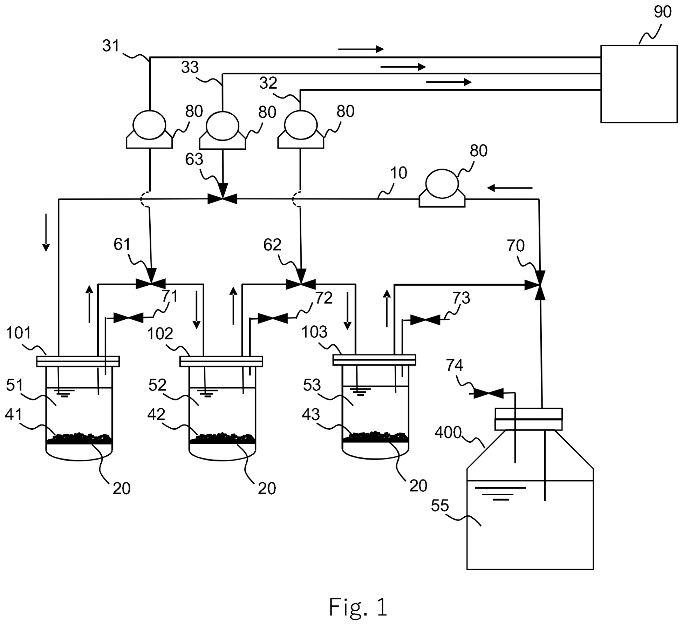

[Fig. 1] Fig. 1 is a figure showing one embodiment of a culture system for obtaining a culture supernatant for culturing muscle cells

### [Embodiments for Carrying Out the Invention]

The purpose, features, advantages, and ideas of the present invention are apparent to those skilled in the art from the description of this specification, and those skilled in the art can easily reproduce the present invention. The embodiments and specific examples of the invention described below show preferred embodiments of the present invention, and are presented for exemplification or explanation, and do not limit the present invention thereto. It is apparent to those skilled in the art that various modifications and alterations can be made based on the description of this specification within the intention and scope of the present invention disclosed in this specification.

### [1] Method for culturing muscle-derived cells

One embodiment of the method for culturing muscle-derived cells according to the present invention is a culture method comprising the step of co-culturing muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

In addition, the muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells may be co-cultured.

Another embodiment of the present invention is a cell culture method comprising the step of circulating a culture medium between a first culture container containing muscle-derived cells and a second culture container containing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, in a culture apparatus in which the first culture container and the second culture container are connected.

Another embodiment of the present invention is a cell culture method comprising the step of culturing muscle-derived cells in the presence of a culture supernatant obtained by culturing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells. The culture supernatant may further contain a culture supernatant obtained by culturing muscle-derived cells. The culture for obtaining the culture supernatant may also be a co-culture.

### [2] Culture Apparatus

### < Culture Apparatus for Culturing Muscle-Derived Cells >

One embodiment of the culture apparatus for culturing muscle-derived cells according to the present invention is a cell culture apparatus comprising one or more first culture containers, each containing muscle-derived cells, and one or more second culture containers, each containing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, the first culture containers and the second culture containers being connected so that the culture medium circulates between the first culture containers and the second culture containers. The culture apparatus may be provided with one or more medium supply containers for supplying a fresh cell culture medium and one or more medium recovery containers for recovering the used cell culture medium. The culture containers are not particularly limited and may be, for example, culture tanks, glass petri dishes, or plastic dishes.

The number of the first culture containers may be one or more than one. When the number of first culture containers is more than one, it is sufficient that at least one of the first culture containers is connected with a certain second culture container for the medium to circulate, but it is preferable that all of the first culture containers are each connected with the certain second culture container for the medium to circulate. The number of second culture containers may be one or more than one. When the number of second culture containers is more than one, it is sufficient that at least one of the second culture containers is connected with a certain first culture container for the medium to circulate, but it is preferable that all of the second culture containers are each connected with the certain first culture container for the medium to circulate. In particular, it is preferable that all of the second culture containers are each independently connected with the certain first culture container for the medium to communicate. When the number of first culture containers is more than one and the number of second culture containers is more than one, it is sufficient that at least one of the first culture containers is connected with at least one of the second culture containers for the medium to circulate, but it is preferable that all of the first culture containers are connected with all of the second culture containers for the medium to circulate.

The culture apparatus may further comprise a third culture container containing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells. The configuration of the above-described second culture container is applied to the configuration of the third culture container. For example, the first to third culture containers may be connected for the medium to circulate, or they may be connected so that the culture medium communicates between the first culture container and the second culture container while independently communicating between the first culture container and the third culture container. Similarly, the type and number of culture containers included in this culture apparatus are not particularly limited.

When the culture containers are connected for the medium to circulate, the culture containers may be directly connected to each other, or they may be connected via one or more culture containers other than the first culture containers and the second culture containers. The one or more culture containers may contain a fresh medium.

The method of connecting the culture containers may be appropriately determined according to the purpose, as long as the first culture containers and the second culture containers are connected so that the culture medium communicates between the first culture containers and the second culture containers, that is, so that the culture medium is shared between the first culture containers and the second culture containers. For example, when there are two culture containers, they may be connected by one communication tube, or they may be connected by two or more communication tubes. By connecting the culture containers with a plurality of communication tubes, the culture medium can be easily circulated. When there are three or more culture containers, they may be connected in series or in parallel, and the culture containers may be connected appropriately according to the purpose to form a suitable culture apparatus.

In addition, the culture containers may be connected by communication tubes, and an open/close control device such as an open/close valve may be provided in the communication tubes between the culture containers, and the communication of the culture medium may be controlled by controlling the opening and closing of the control device. A pump may also be provided in the communication tubes between the culture containers to promote the flow of the culture medium in either direction.

### < Culture System >

The term "culture system" refers to a system in a culturing state, including a culture apparatus, a cell culture medium, and cultured cells. The cell culture medium may be a serumcontaining medium, but it is preferably a serum-free medium in terms of ease of controlling the contained substances. A person skilled in the art can select a cell culture medium suitable for the cells to be cultured.

In the case of co-culture, a plurality of types of cells may be cultured in one culture container, in which case the plurality of types of cells may be mixed and cultured, or the culture container may be divided into a plurality of compartments, and a single type of cells may be cultured in each compartment. A separate culture container may be provided for each type of cells, and the cells may be cultured separately in the respective culture containers by connecting the culture containers and thus sharing the culture medium so that the culture medium communicates between or among the culture containers and becomes uniform as a whole.

In the case that the culture is carried out by adding one or more culture supernatants to the cells for proliferation, the culture container for the cells for proliferation may be separate from one or more cell containers for the cells for obtaining the culture supernatants. The number of the cell containers for the cells for obtaining the culture supernatants may be one or more than one, regardless of the number of types of cells used, but it is preferable that one or more cell containers are provided for each type of cells. These one or more cell containers may be separate or may be appropriately connected according to the purpose. It is preferable that the cells for obtaining the culture supernatants include the same type of cells as the cells for proliferation.

The culture container for each type of cells may be one or more than one. In any case, the culture containers can be appropriately connected according to the purpose to form a suitable culture system.

### [3] Specific Method for Preparing and Culturing Cells

### < Origin of Cells >

The animal for obtaining muscle is not particularly limited, but is preferably a vertebrate such as a mammal, and more preferably an animal that lays eggs with an eggshell, for example, a bird or a reptile. The biological species from which the skin-derived cells, intestine-derived cells, heart-derived cells, brain-derived cells, stomach-derived cells, embryonic membrane-derived cells, liver-derived cells, kidney-derived cells, and lung-derived cells are derived is not particularly limited, but is preferably a vertebrate, and may be any of a mammal, a reptile, a bird, or an amphibian, and for example, may be a human, a cow, a pig, a rabbit, or a chicken. In addition, the biological species from which the embryonic membrane-derived cells are derived is not particularly limited as long as it is oviparous, and may be a bird or a reptile, and may be a chicken. In the case of a chicken, it may be a 0-day to 21-day embryo, a 2-day to 19-day embryo, a 4-day to 17-day embryo, or a 6-day to 15-day embryo. The cells of each tissue may be derived from the same animal or from different animals.

### < Cell Types and Preparation Method of Cells >

The muscle-derived cells, skin-derived cells, intestine-derived cells, heart-derived cells, brain-derived cells, stomach-derived cells, embryonic membrane-derived cells, liver-derived cells, kidney-derived cells, and lung-derived cells can each be obtained by using muscle, skin, intestine, heart, brain, stomach, embryonic membrane, liver, kidney, and lung taken from animal embryos, or by using whole embryos, and separating cells by chemical treatment such as with enzymes including collagenase, dispase, or trypsin, or by physical treatment such as cutting or crushing with a scalpel or razor, or pipetting using a microchip. Specific methods have already been established as common technical knowledge, and as long as the method enables the cells to be collected alive, it is not particularly limited.

The muscle-derived cells include one or more types of cells selected from the group consisting of muscle cells, nerve cells, connective tissue cells, fibroblasts, vascular endothelial cells, vascular pericytes, blood cells, and precursor cells or stem cells thereof. The muscle may be skeletal muscle, smooth muscle, or cardiac muscle, or a mixture of two or more thereof.

The skin-derived cells include epithelial cells and dermal cells.

The intestine-derived cells include one or more types of cells selected from the group consisting of intestinal epithelial cells, CBC cells, Paneth cells, enteroendocrine cells, goblet cells, absorptive epithelial cells, M cells, intestinal wall cells, connective tissue cells, muscle cells, fibroblasts, vascular endothelial cells, vascular pericytes, and precursor cells or stem cells thereof. The intestine may be the duodenum, small intestine, large intestine, or colon, or a mixture of two or more thereof.

The heart-derived cells include one or more types of cells selected from the group consisting of myocardial cells, nerve cells, connective tissue cells, fibroblasts, vascular endothelial cells, vascular pericytes, blood cells, and precursor cells or stem cells thereof.

The brain-derived cells include one or more types of cells selected from the group consisting of nerve cells, glial cells, pituitary cells, vascular endothelial cells, vascular pericytes, and precursor cells or stem cells thereof.

The stomach-derived cells include one or more types of cells selected from the group consisting of chief cells, parietal cells, mucous neck cells, and precursor cells or stem cells thereof.

The embryonic membrane-derived cells include one or more types of cells selected from the group consisting of amnion, serosa, allantois, and yolk sac.

The liver-derived cells include one or more types of cells selected from the group consisting of parenchymal cells (i.e., hepatocytes), non-parenchymal cells (for example, sinusoidal wall cells, myofibroblasts, bile duct epithelial cells, connective tissue cells, hepatic stellate cells, etc.), and precursor cells or stem cells thereof.

The kidney-derived cells include one or more types of cells selected from the group consisting of: epithelial cells, endothelial cells, mesangial cells, visceral epithelial cells, and parietal epithelial cells, constituting the glomerulus; endothelial cells and epithelial cells constituting the renal tubule; connective tissue cells, fibroblasts, vascular endothelial cells, vascular pericytes, filling the remaining tissue; and precursor cells or stem cells thereof.

The lung-derived cells include one or more cells selected from the group consisting of endothelial cells, alveolar epithelial cells, and precursor cells or stem cells thereof.

The cells may be primary cultured cells or established cultured cell lines. The cells may be cultured in the form of a tissue or organ, in the form of a cell mass, or as individually separated cells.

The culture may be a suspension culture or an adhesion culture. The culture may be a planar culture or a three-dimensional culture. The selection of culture to be used depending on the type of cells can be easily and appropriately determined by a person skilled in the art.

The specific cell culture method can be selected by a person skilled in the art using the above-described culture system in a manner suitable for the cells to be cultured.

When a culture supernatant obtained by culturing other types of cells is used in the culture, the culture supernatant can be collected, for example, after seeding the cells into a cell container at a density of 1×10⁴ to 1×10⁷ cells/mL and culturing for about 1 to 30 days. Thereafter, centrifugation may be performed to remove precipitates such as cell debris. The obtained culture supernatant may be used as is (that is, with 100% cell supernatant) to culture the cells for proliferation, or a medium obtained by adding fresh medium at 10% or more, 30% or more, 50% or more, or 70% or more may be used. When culture supernatants of two or more types of cells are added to the medium for the cells for proliferation, the two or more types of cells for obtaining the culture supernatants may be cultured separately and the supernatants mixed, or some or all of the cells may be co-cultured. **In** the case of co-culture, some or all of the co-cultured cells may be cultured in one culture container, or separate culture containers may be provided for each cell type and the cells may be cultured separately by sharing the medium. **In** the latter case, the culture containers may be connected so that the medium communicates between the culture containers. The method of connecting the culture containers may be appropriately determined according to the purpose. For example, some or all of the culture containers may be connected in a ring shape, and a culture container of the same type of cell as the cell to be proliferated may be included therein.

### < Culture System for Obtaining Culture Supernatant >

An example of a culture system for obtaining a culture supernatant using a culture apparatus including culture containers and tubes connecting the containers is shown below with reference to Fig. 1; however, the embodiment of the culture system is not limited thereto, and various improvements and modifications can easily be made by a person skilled in the art.

This culture system includes a plurality of culture containers 101 to 103 containing scaffolds 20 for cell culture, cultured cells 41 to 43, and culture supernatants 51 to 53. The cultured cells 41 to 43 cultured in each culture container 101 to 103 may be of the same type or different types, and may include a cell type for proliferation using the obtained supernatant. The culture containers 101 to 103 are connected by communication tubes 10 so that the culture supernatants 51 to 53 can circulate, and three-way valves 61 to 63 are provided between the containers 101 to 103. Recovery tubes 31 to 33 are provided so that the culture supernatant can be recovered into a recovery container 90 by controlling the three-way valves 61 to 63 from the system of the medium circulation. A medium container 400 containing fresh medium 55 is connected to the circulation system of the medium using a three-way valve 70. A liquid feed pump 80 is provided in the communication tubes 10 and recovery tubes 30. The culture containers 101 to 103 and the medium container 400 are provided with tubes that open to the outside air and are provided with open/close valves 71 to 73 and 74, and filters are provided in the tubes so that microorganisms and small particles do not enter from the outside air.

By using such a device, it is possible to recover a supernatant that meets the aim. For example, by opening the valve 61 only in the direction from the culture container 101 to the recovery container 90 and opening the valves 63 and 70 only in the direction from the medium container 400 to the culture container 101, a culture supernatant cultured only with the cultured cells 41 can be obtained. Alternatively, by opening the valves 61 to 63 and valve 70 only in directions such that the medium circulates, a uniform culture supernatant obtained by the co-culture of the cultured cells 41 to 43 circulates through the circulation system. Thereafter, by opening valve 71 and opening valve 63 only in the direction from the container 103 to the recovery container 90, the uniform culture supernatant can be recovered. Thereafter, by closing valve 71, opening valves 73 and 74, opening valves 61 to 63 only in directions such that the medium circulates, and opening valve 70 only in the direction from the medium container 400 to the container 101, fresh medium is supplied to the culture containers 101 to 103. Alternatively, by appropriately combining the opening and closing of each valve, the amount of medium in each culture container 101 to 103 and the combination of recovered media can be freely controlled.

Thus, the supernatant suitable for the purpose can be collected in the recovery container 90, and the recovered supernatant can be used for culturing cells for proliferation. In addition, the recovery container 90 and the culture container for the cells for proliferation may be connected so that the supernatant collected in the recovery container 90 is automatically supplied to the culture container for the cells for proliferation. In this case, the supply amount, supply frequency, and number of supplies of the supernatant may be controlled by computer.

### [Examples]

### [1] Method for Preparing Cells

The cells were prepared from each tissue (muscle, skin, stomach, heart, brain, intestine, embryonic membrane, liver, kidney, and lung) as follows.

First, fertilized chicken eggs were incubated, and the tissues were each collected from 12-day embryos. The tissues were dispersed into single cells by physical treatment using a mesh (100 µm), and the dispersed cells were collected.

### [2] Method for Culturing Cells

The collected cells were seeded together with scaffolds into three cell culture plates connected by communication tubes, using the cells #1 to #3 shown in Table 1, and cultured at 37°C under 5% CO₂ using DMEM supplemented with 0.05% yeast extract as the medium. On day 6 of culture, 50% of the medium was exchanged, and on day 12 of culture, the cells were collected and the cell number was measured. The relative value of the obtained cell number was calculated with the cell number obtained by culturing each single type of cells under the same conditions separately from the plates #1 to #3 as 1. The experiment was repeated nine times, and Table 1 shows the mean value ± standard deviation.

### [3] Results

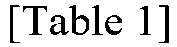

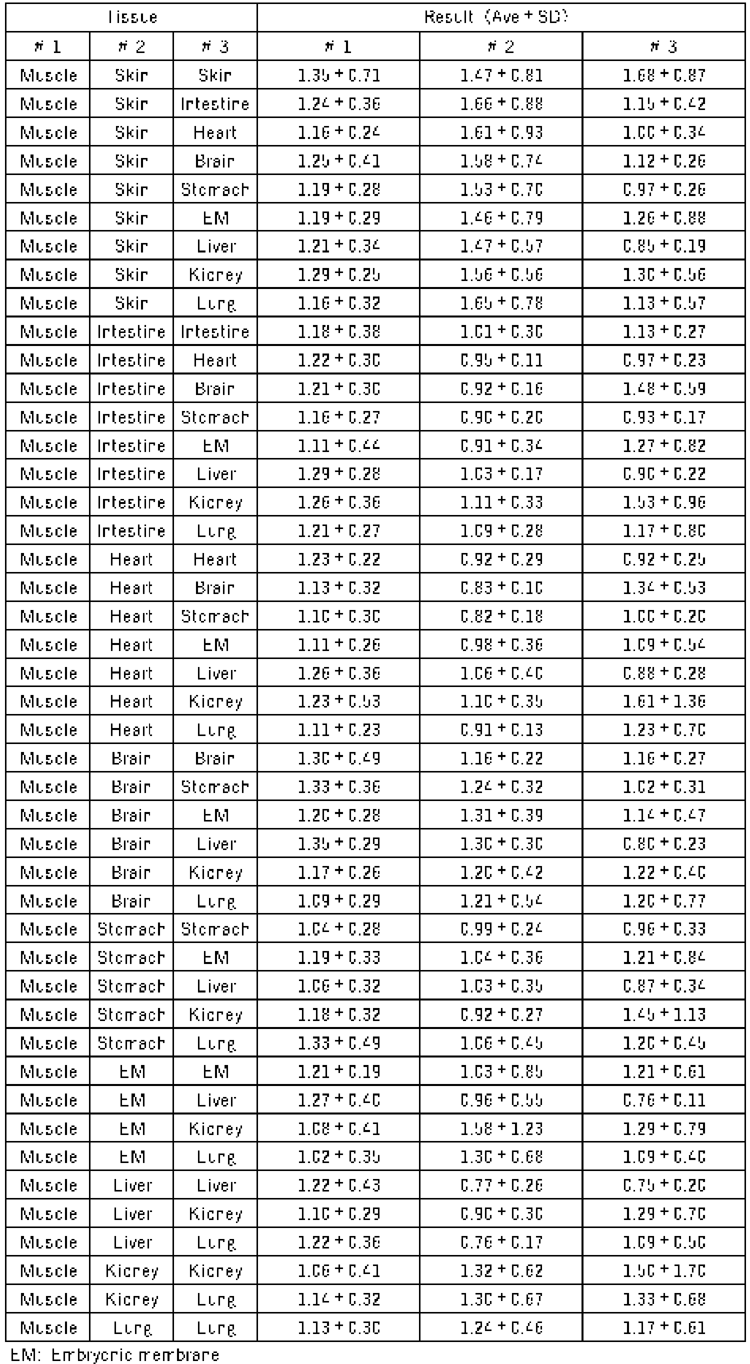

As shown in Table 1, by culturing muscle cells in a medium to which the supernatants of various cell types are added, it becomes possible to promote proliferation. **In** particular, when co-cultured with a combination of cells selected from the group consisting of:
(1) intestine cells, and cells selected from the group consisting of liver cells, kidney cells, heart cells, brain cells, and lung cells,
(2) skin cells, and cells selected from the group consisting of kidney cells, brain cells, intestine cells, and liver cells,
(3) brain cells, and liver cells or stomach cells,
(4) heart cells, and kidney cells or liver cells,
(5) embryonic membrane cells and liver cells,
(6) liver cells and lung cells,
(7) stomach cells and lung cells,
or when co-cultured with one type of cells selected from the group consisting of skin cells, brain cells, heart cells, embryonic membrane cells, and liver cells, the proliferation of muscle cells was remarkably promoted.

### [Industrial Applicability]

According to the present invention, it is possible to provide a method for culturing muscle-derived cells and a culture apparatus containing muscle-derived cells.

## Claims

1. A cell culture method comprising the step of co-culturing muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

2. A cell culture method comprising the step of circulating a culture medium between a first culture container and a second culture container in a culture apparatus, the first culture container and the second culture container being connected, the first culture container comprising muscle-derived cells, the second culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

3. The cell culture method according to claim 2, comprising the step of circulating the culture medium among the first culture container, the second container and a third container in the culture apparatus, the first culture container, the second container and the third container being connected, the first culture container comprising the muscle-derived cells, the second culture container comprising the at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, the third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

4. The cell culture method according to claim 2, comprising the step of communicating the culture medium between the first culture container and the second culture container and the step of communicating the culture medium between the first culture container and a third culture container in the culture apparatus, wherein the first culture container comprising the muscle-derived cells, the second culture container comprising the at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, and the third culture container comprising at least one type of cells selected from the same group are connected.

5. A cell culture apparatus comprising a first culture container and a second culture container, the first culture container and the second culture container being connected so that a culture medium is circulated between the first culture container and the second culture container, the first culture container comprising muscle-derived cells, the second culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

6. The cell culture apparatus according to claim 5, further comprising a third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, wherein the first to third culture containers are connected so that the culture medium circulates among the first to third culture containers.

7. The cell culture apparatus according to claim 5, further comprising a third culture container comprising at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells, wherein the first culture container and the second culture container are connected so that the culture medium communicates between the first culture container and the second culture container, and the first culture container and the third culture container are connected so that the culture medium communicates between the first culture container and the third culture container.

8. A culture system for co-culturing muscle-derived cells and at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

9. A cell culture method comprising the step of culturing muscle-derived cells in the presence of a culture supernatant obtained by culturing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

10. The cell culture method according to claim 9, wherein the culture supernatant further comprises a culture supernatant obtained by culturing muscle-derived cells.

11. The cell culture method according to claim 9 or 10, wherein the culture for obtaining the culture supernatant is a co-culture.

12. A culture system for culturing muscle-derived cells in the presence of a culture supernatant obtained by culturing at least one type of cells selected from the group consisting of skin-derived cells, intestine-derived cells, heart-derived cells, brain-derived cells, stomach-derived cells, embryonic membrane-derived cells, liver-derived cells, kidney-derived cells, and lung-derived cells.

13. The culture system according to claim 12, wherein the culture supernatant further comprises a culture supernatant obtained by culturing muscle-derived cells.

14. The culture system according to claim 12 or 13, wherein the culture for obtaining the culture supernatant is a co-culture.
